# FASCICULE DE BREVET EUROPEEN

(11) **EP 1 211 981 B1**
(45) Date de publication et mention de la délivrance du brevet: **10.12.2008**
(21) Numéro de dépôt: 00958759.3
(22) Date de dépôt: 29.08.2000
(51) Int. Cl.: A61B 6/14

(54) **PROCEDE POUR OBTENIR UNE IMAGE RADIOGRAPHIQUE D'UNE DENT ET DE SON ENVIRONNEMENT, ET DISPOSITIFS PERMETTANT DE METTRE EN OEUVRE CE PROCEDE**
RÖNTGENABBILDUNGSVERFAHREN EINES ZAHNES UND SEINER UMGEBUNG, UND GERÄT ZUR UMSETZUNG DIESES VERFAHRENS
METHOD FOR OBTAINING A RADIOGRAPHIC IMAGE OF A TOOTH AND ITS SURROUNDING ENVIRONMENT, AND DEVICES IMPLEMENTING SAID METHOD

(30) Priorité: 30.08.1999 FR 9910911
(43) Date de publication de la demande: 12.06.2002
(73) Titulaire: Carestream Health, Inc., Rochester, New York 14608 (US)
(72) Inventeur: MOUYEN, Francis, Pas-de-la-Case Principauté d'Andorre (AD)
(74) Mandataire: Santarelli
(86) Numéro de dépôt international: PCT/FR2000/002398
(87) Numéro de publication internationale: WO 2001/015603

(56) Documents cités:
- EP-A- 0 357 944
- EP-A- 0 413 043
- EP-A- 0 756 416
- DE-A- 4 441 939
- US-A- 5 434 418

## Description

L'invention a trait aux techniques de radiologie dentaire et concerne plus particulièrement les procédés pour obtenir une image radiographique d'une dent et de son environnement, ainsi que les dispositifs permettant de mettre en oeuvre ces procédés.

L'évolution de l'électronique observée ces dernières années a permis de faire progresser notablement les techniques d'examens radiologiques des organes du corps humain. Cette évolution vise plus particulièrement à réduire, pour le patient et pour le manipulateur, les doses d'exposition aux rayons X tout en améliorant la qualité d'image de la cible radiographiée. Les procédés et les dispositifs décrits et représentés dans les brevets français publiés sous les numéros 2 333 404, 2 378 496, 2 415 938, 2 495 429, 2 476 949, 2 477 626, 2 479 636, 2 185 667, 2 247 749 et 2 310 059 démontrent bien cet état de fait.

Les techniques d'exploration dentaire en sont, quant à elles, restées au stade traditionnel de la radiographie qui consiste à intercaler la dent à examiner entre une source extra-buccale de rayons X et un film radiographique intra-buccal sensible aux rayons X traversant la dent irradiée. Les formes de l'image obtenue sur ce film radiographique correspondent aux ombres portées par les constituants plus ou moins opaques aux rayons X de la dent examinée. Bien que cette technique de radiographie dentaire soit la plus usitée à l'heure actuelle, elle présente toutefois l'inconvénient de limiter le nombre de clichés, compte tenu des doses de rayons X qu'ils exigent.

Il est utile de préciser que les nouvelles techniques de radiologie ont plus particulièrement porté sur la réalisation intrinsèque du capteur du faisceau de rayons X émergeant de la cible irradiée afin, comme on l'a rappelé ci-dessus, de réduire les temps d'exposition aux rayons X tout en améliorant la qualité d'image de la cible radiographiée. De plus, l'image est obtenue en temps réel, évitant ainsi les manipulations de développement du film de la radiographie dentaire traditionnelle.

On connaît déjà de tels appareils, par exemple celui qui est décrit et représenté dans le brevet américain n° 4 160 997 (SCHWARTZ).

Le capteur intra-buccal décrit dans ce brevet américain présente cependant de nombreux inconvénients, en particulier l'inconvénient majeur de ne pas pouvoir remplir sa fonction essentielle qui consiste à enregistrer le faisceau de rayons X émergeant d'une dent irradiée et à fournir en sortie des informations susceptibles d'être analysées par une unité de traitement électronique, ceci afin de faire apparaître, sur le moniteur d'une chaîne de visualisation, l'image de la susdite dent. En effet, pour comprendre que ce capteur intra-buccal n'est pas fonctionnel, il est utile de rappeler que les dispositifs à transfert de charges présentent les particularités suivantes:
- les dimensions de leur face sensible ne sont pas suffisantes pour capter tous les rayons X d'un faisceau émergeant d'une dent irradiée et SCHWARTZ propose d'utiliser, dans son capteur intra-buccal, un écran assurant une transmission linéaire,
- leur face sensible se détériore sous l'impact de rayons X d'énergie supérieure à 1 KeV et l'écran de SCHWARTZ n'assure par une protection suffisante de cette face sensible,
- l'unité de traitement électronique des informations électriques en sortie du dispositif à transfert de charges ne doit pas être éloignée de ce dernier de plus de vingt centimètres, distance limite au-delà de laquelle le signal de sortie est trop faible pour être traité, et l'unité de traitement électronique des informations en sortie du capteur intra-buccal de SCHWARTZ est extra-buccale et reliée à ce dernier par un câble d'une longueur de plus de vingt centimètres.

Faisant le bilan de cet état de fait, le demandeur a mené des recherches qui ont abouti à la réalisation d'un appareil permettant d'obtenir une image radiologique dentaire sur un moniteur d'une chaîne de visualisation et d'obvier aux inconvénients précités, pour offrir un appareil fonctionnel aux performances incontestables dans la qualité de reproduction de l'image dentaire et dans la réduction de la dose d'exposition aux rayons X. Il a déjà déposé une demande de brevet dans ce domaine, la demande EP 0 129 451.

Le document US 5,434,418 se rapporte à un capteur intra-buccal très mince pour radiographie assistée par ordinateur, résistant à l'humidité, à la chaleur et pouvant être autoclavé. Le capteur selon ce document comporte un dispositif CCD ou photodiode revêtu d'une épitaxie fine d'un matériau tel que du iodure de césium dopé au thallium, agencé en barreau afin de le protéger des rayons X et de convertir ces derniers en lumière. Le capteur revêtu est collé à un support céramique et possède un amplificateur de signal intégré. Le capteur selon ce document ne comporte pas d'élément optique, le dispositif CCD étant directement lié au barreau de iodure de césium. De ce fait, pour protéger efficacement la couche CCD contre les rayons X la couche d'iodure de césium doit être suffisamment épaisse (entre 200 et 300 mm) pour transformer un maximum de rayons X en photons ; cependant et dans ces conditions, cette épaisseur de couche d'iodure de césium entraîne un effet secondaire qui est de freiner et de s'opposer à la transmission des photons et limite donc la quantité de lumière qui parvient jusqu'au dispositif CCD ; ce qui provoque une réduction de rendement du capteur.

Le demandeur a continué ses études pour améliorer ces appareils, notamment en vue d'augmenter leur sensibilité, leur fiabilité, etc.

La présente invention est le résultat actuel de ses études dans la domaine. Elle a pour but de perfectionner les procédés connus de l'art antérieur pour obtenir une image radiographique d'une dent et de son environnement, ainsi que les dispositifs permettant de mettre en oeuvre ces procédés.

Plus précisément, la présente invention a pour objet un procédé pour obtenir une image radiographique d'une dent et de son environnement, caractérisé par le fait qu'il consiste:
à émettre un faisceau de rayons X en direction de ladite dent et de son environnement,
à guider les rayons X émergeant de ladite dent et de son environnement, dans des volumes sensiblement cylindriques suivant sensiblement l'axe desdits volumes,
à transformer les rayons X lors de leur guidage dans lesdits volumes cylindriques, en rayons lumineux de longueur d'onde supérieure à celle des rayons X,
à guider lesdits rayons lumineux dans un faisceau de fibres optiques,
à convertir ces rayons lumineux en signaux électriques, et
à traiter ces signaux électriques pour réaliser ladite image radiographique.

La présente invention a aussi pour objet un dispositif pour mettre en oeuvre le procédé ci-dessus qui est conforme à la revendication 5.

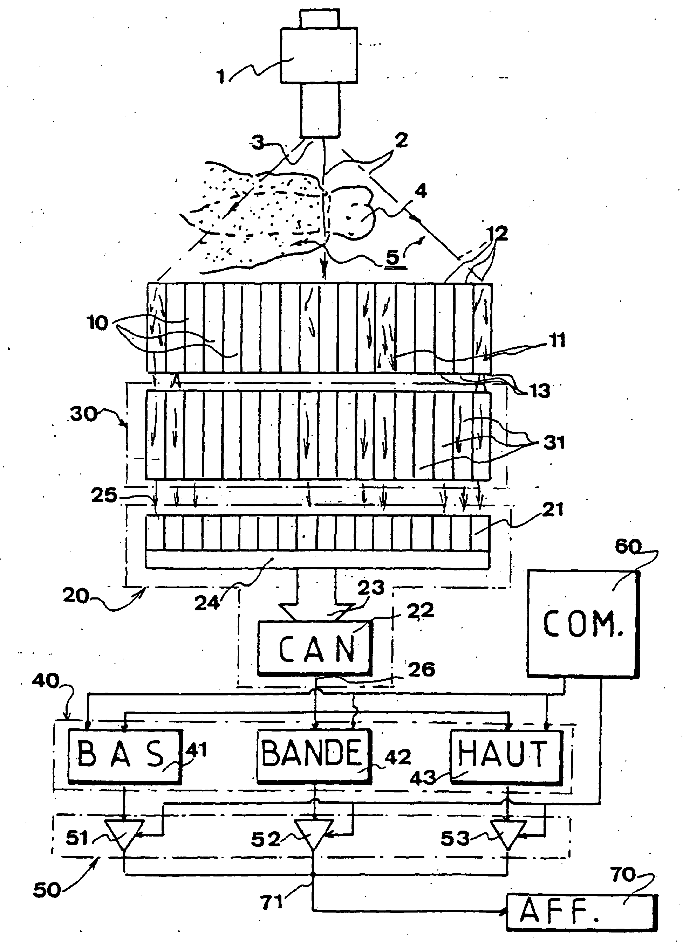

D'autres caractéristiques et avantages de la présente invention apparaîtront au cours de la description suivante donnée en regard du dessin annexé à titre illustratif, mais nullement limitatif, dans lequel:

La figure unique représente le schéma de principe d'un dispositif selon l'invention pour obtenir une image radiographique d'une dent et de son environnement.

La présente invention concerne un procédé pour obtenir une image radiographique d'une dent et de son environnement, c'est-à-dire la partie de gencive dans laquelle est implantée la dent, éventuellement même l'os du maxillaire, les éventuelles cavités que peut comporter une telle dent, remplies ou non d'un amalgame ou analogue, etc.

Le procédé consiste essentiellement à émettre un faisceau de rayons X en direction de la dent et de son environnement. Les rayons X émergeant de la dent et de son environnement sont guidés dans des volumes sensiblement cylindriques suivant sensiblement l'axe de ces volumes, tout en étant transformés en rayons lumineux de longueur d'onde très supérieure à celle des rayons X et choisie de façon à permettre la conversion de ces rayons lumineux en signaux électriques.

Le procédé consiste enfin à traiter ces signaux électriques pour réaliser l'image radiographique, par exemple sous forme d'image vidéo ou analogue.

Dans une mise en oeuvre préférentielle, le procédé comporte en outre une étape qui consiste à filtrer les signaux électriques en fonction de critères prédéterminés, par exemple, mais non limitativement, pour éliminer les signaux électriques qui correspondent aux images des tissus mous de la gencive ou à des matières comme les amalgames ou analogues, dans le but de ne conserver que les signaux électriques qui correspondent aux images de la dentine:

Ces derniers signaux peuvent ensuite être amplifiés selon une fonction prédéterminée, linéaire ou non, pour avoir par exemple une partie seulement de l'image de la dentine dilatée de façon que le praticien dentiste puisse effectuer une analyse plus précise de l'état de la dent radiographiée.

Le procédé dont les étapes ont été décrites ci-dessus est avantageusement mis en oeuvre avec un dispositif dont le schéma de principe est représenté sur la figure unique.

Le dispositif schématiquement illustré sur la figure unique comporte une source 1 de rayons X apte à émettre un faisceau 2 de rayons X à partir d'une fenêtre de sortie 3. Cette source 1 de rayons X est apte à être positionnée de façon que sa fenêtre de sortie soit dirigée vers une dent 4 et son environnement 5.

Le dispositif comporte aussi une pluralité de barreaux cylindriques 10 réalisés en un matériau apte à transformer les rayons X en rayons lumineux 11 d'une longueur d'onde supérieure à celle des rayons X.

Chaque barreau comporte une face d'entrée 12 apte à recevoir les rayons X du faisceau 2 après qu'ils aient traversé la dent 4 et son environnement 5, et une face de sortie 13 apte à émettre les rayons lumineux 11. Ces barreaux cylindriques 10 sont disposés les uns à côté des autres de façon que toutes les faces d'entrée 12 soient tournées vers la fenêtre de sortie 3 de la source 1 de rayons X.

Dans une réalisation avantageuse, les barreaux cylindriques 10 sont réalisés dans un cristal de iodure de césium et sont sensiblement cylindriques de révolution, d'une longueur comprise entre 80 et 200 µm, de préférence entre 100 et 120 µm, pour un diamètre compris entre 3 et 7 µm, de préférence entre 4 et 6 µm.

En outre, il est certainement, avantageux que, comme illustré sur la figure unique, ces barreaux cylindriques soient disposés au contact les uns des autres pour former en quelque sorte une mosaïque dont l'épaisseur est égale à la longueur d'un barreau.

Le dispositif selon l'invention présente un avantage essentiel par rapport aux dispositifs antérieurs du même type : la structure et la disposition des barreaux cylindriques 10 telles que décrites ci-dessus permettent de guider parfaitement les rayons X qui pénètrent dans ces barreaux par leur face d'entrée 12. Il ne se produit qu'une très faible dispersion de rayons X dans l'espace environnant, ce qui permet de transformer en rayons lumineux la quasi totalité des rayons X ayant traversé la dent et son environnement et ainsi d'augmenter très notablement la sensibilité du dispositif par rapport aux dispositifs du même type connus de fart antérieur.

Le dispositif comporte en outre des moyens 20 pour convertir les rayons lumineux 11 en signaux électriques.

Dans une réalisation préférentielle, ces moyens 20 comportent un convertisseur 21 de rayons lumineux en signaux électriques analogiques et un convertisseur 22 de signaux électriques analogiques en signaux électriques numériques dont l'entrée 23 est reliée à la sortie 24 du convertisseur analogique 21.

Avantageusement, le convertisseur analogique 21 est constitué par une barrette de CCD, tandis que le convertisseur numérique 22 est un CAN du type à au moins douze bits. De manière alternative, le convertisseur analogique peut être constitué d'une barrette de CMOS.

Bien entendu, le dispositif comporte des moyens 30 pour coupler les faces de sortie 13 des barreaux cylindriques 10 avec les faces photosensibles 25 du convertisseur analogique 21. Ces moyens de couplage 30 peuvent être constitués par des moyens pour positionner les faces photosensibles 25 en regard des faces de sortie 13 des barreaux. Cependant, il peut être avantageux, dans un but dé concevoir un dispositif facilement manipulable, que ces moyens de couplage 30 soit constitués par un faisceau de fibres optiques, comme schématiquement illustré en 31 sur la figure, les faces d'entrée des fibres optiques étant positionnées en regard des faces de sortie 13 des barreaux et leurs faces de sortie étant disposées en regard des faces photosensibles 25 du convertisseur 21.

Avantageusement, afin de rendre l'utilisation du dispositif plus adaptée au désir des praticiens dentistes, le dispositif comporte des moyens commandables 40 pour filtrer les signaux électriques obtenus en sortie des moyens 20 définis ci-avant, c'est-à-dire, dans le mode de réalisation schématiquement illustré sur la figure, en sortie 26 du convertisseur numérique CAN 22.

Le dispositif peut compter un seul filtre dont la bande passante peut être adaptée aux souhaits du praticien.

Comme illustré, ce filtre peut être avantageusement équivalent à un ensemble, par exemple, de trois filtres. A titre d'exemple: un filtre "passe-bas" 41, un filtre "passe-bande" 42 et un filtre "passe-haut" 43.

Le filtre passe-bas 41 est par exemple apte à éliminer les signaux électriques engendrés par les rayons X ayant traversé les parties les plus opaques de la dent 4 et de son environnement 5, comme une cavité de la dent remplie d'un amalgame ou analogue, un pivot planté dans une racine, etc.

Le filtre passe-bande 42 est par exemple apte à ne laisser passer que les signaux électriques engendrés par les rayons X ayant traversé la dentine de la dent 4 et les parties de matière de son environnement 5 sensiblement équivalentes à la dentine.

Le filtre passe-haut 43 est par exemple apte à éliminer les signaux électriques engendrés par les rayons X ayant traversé les tissus mous ou équivalents de la dent et de son environnement, par exemple la chair de la gencive.

De manière alternative, lesdits moyens 40 pour filtrer les signaux électriques comportent au moins l'un des trois filtres suivants: un filtre passe-bas 41 apte à éliminer les signaux électriques correspondant aux rayons X ayant traversé les parties les plus opaques de ladite dent 4 et de son environnement 5, un filtre passe-bande 42 apte à laisser passer les signaux électriques correspondant aux rayons X ayant traversé la dentine de la dent et les parties de matière de son environnement sensiblement équivalentes à cette dentine, un filtre passe-haut 43 apte à éliminer les signaux électriques correspondant aux rayons X ayant traversé des tissus mous ou équivalents de la dent et de son environnement.

En outre, dans une réalisation avantageuse, le dispositif comporte des moyens 50 pour amplifier les signaux électriques délivrés par les moyens 20 de conversion des rayons lumineux 11 en signaux électriques.

Dans l'exemple illustré, ces moyens d'amplification 50 sont constitués par trois amplificateurs commandables 51, 52, 53 dont les entrées sont respectivement connectées aux sorties des trois filtres 41, 42, 43.

De cette façon, les signaux obtenus à la sortie 44 des moyens de filtre 40 peuvent être amplifiés selon une fonction prédéterminée, par exemple linéaire pour obtenir une image homothétique, sans distorsion, de l'image radiographique de la dent 4 et de son environnement 5. Mais il peut être avantageux de choisir une fonction non linéaire de façon à obtenir un effet de loupe sur une partie choisie de l'image radiographique, dans le but d'effectuer une analyse plus précise de l'état de cette partie.

Bien entendu, l'ensemble des trois filtres 41-43 et des trois amplificateurs 51-53 est avantageusement piloté par une unité de commande 60 du type ordinateur ou analogue.

Enfin, le dispositif comporte des moyens 70 pour traiter les signaux électriques obtenus, dans l'exemple illustré, en sortie 71 des amplificateurs 51-53, en vue de réaliser i'image radiographique de la dent et de son environnement. Ces moyens 70 de traitement de signaux électriques peuvent être de différents types, par exemple constitués de mémoires temporaires ou permanentes, ou généralement d'un convertisseur de signaux électriques en signaux vidéo aptes à être visualisés sur l'écran d'un moniteur.

## Revendications

1. Procédé pour obtenir une image radiographique d'une dent et de son environnement, consistant:
à émettre un faisceau de rayons X en direction de ladite dent et de son environnement,
à guider les rayons X émergeant de ladite dent et de son environnement, dans des volumes sensiblement cylindriques, suivant sensiblement l'axe desdits volumes,
à transformer les rayons X lors de leur guidage dans lesdits volumes cylindriques, en rayons lumineux de longueur d'onde supérieure à celle des rayons X,
à guider lesdits rayons lumineux dans un faisceau de fibres optiques,
à convertir ces rayons lumineux en signaux électriques, et
à traiter ces signaux électriques pour réaliser ladite image radiographique.

2. Procédé selon la revendication 1, ***caractérisé par* le *fait qu***'il consiste en outre à filtrer lesdits signaux électriques en fonction de critères prédéterminés.

3. Procédé selon l'une des revendications précédentes, ***caractérisé par le fait que*** lesdits rayons lumineux sont convertis en signaux électriques analogiques et que ces dits signaux électriques analogiques sont convertis en signaux électriques numériques.

4. Procédé selon l'une des revendications précédentes, ***caractérisé par le fait qu***'il consiste à amplifier au moins une partie desdits signaux électriques suivant une fonction prédéterminée.

5. Dispositif pour mettre en oeuvre le procédé selon l'une des revendications précédentes, comportant :
une source (1) apte à émettre un faisceau de rayons X vers ladite dent (4) et son environnement (5),
un barreau réalisé en un matériau apte à transformer les rayons X en rayons lumineux de longueur d'onde supérieure à celle des rayons X, le barreau comportant une face d'entrée apte à recevoir lesdits rayons X et une face de sortie apte à émettre lesdits rayons lumineux.
des moyens (20) pour convertir des rayons lumineux (11) en signaux électriques,
des moyens (30) pour coupler la face de sortie (13) du barreau cylindrique avec lesdits moyens (20) pour convertir des rayons lumineux (11) en signaux électriques, et
des moyens (70) pour traiter lesdits signaux électriques en vue de réaliser ladite image radiographique,
**caractérisé par le fait que** le dispositif comporte une pluralité de barreaux cylindriques (10) réalisés en un matériau apte à transformer les rayons X en rayons lumineux (11) de longueur d'onde supérieure à celle des rayons X, chaque barreau comportant une face d'entrée (12) apte à recevoir lesdits rayons X et une face de sortie (13) apte à émettre lesdits rayons lumineux (11), lesdits barreaux cylindriques (10) étant disposés les uns à côtés des autres de façon que toutes les faces d'entrée (12) soient tournées vers ladite source (1) de rayons X,
les moyens (30) pour coupler les faces de sortie (13) des barreaux cylindriques avec lesdits moyens (20) pour convertir des rayons lumineux en signaux électriques comportant un faisceau de fibres optiques.

6. Dispositif selon la revendication 5, ***caractérisé par le fait que*** les moyens (20) pour convertir des rayons lumineux en signaux électriques comportent un convertisseur (21) de rayons lumineux en signaux électriques analogiques et un convertisseur (22) de signaux électriques analogiques en signaux électriques numériques dont l'entrée (23) est reliée à la sortie (24) du convertisseur de rayons lumineux en signaux électriques analogiques.

7. Dispositif selon la revendication 6, ***caractérisé par le fait que*** ledit convertisseur (21) de rayons lumineux en signaux électriques analogiques est constitué par une barrette de CCD et que ledit convertisseur (22) de signaux électriques analogiques en signaux électriques numériques est un convertisseur CAN du type à au moins douze bits.

8. Dispositif selon l'une des revendications 5 à 7, ***caractérisé par* le *fait qu***'il comporte en outre des moyens (40) pour filtrer lesdits signaux électriques.

9. Dispositif selon la revendication 8, **caractérisé *par le fait que*** lesdits moyens (40) pour filtrer les signaux électriques comportent au moins l'un des trois filtres suivants: un filtre passe-bas (41) apte à éliminer les signaux électriques correspondant aux rayons X ayant traversé les parties les plus opaques de ladite dent (4) et de son environnement (5), un filtre passe-bande (42) apte à laisser passer les signaux électriques correspondant aux rayons X ayant traversé la dentine de la dent et les parties de matière de son environnement sensiblement équivalentes à cette dentine, un filtre passe-haut (43) apte à éliminer les signaux électriques correspondant aux rayons X ayant traversé des tissus mous ou équivalents de la dent et de son environnement.

10. Dispositif selon la revendication 9, ***caractérisé par le fait qu***'il comporte en outre des moyens (50) pour amplifier les signaux électriques obtenus à la sortie (44) d'au moins l'un des trois filtres (41-43), selon une fonction prédéterminée.

11. Dispositif selon l'une des revendications 5 à 10, ***caractérisé par le fait que*** les moyens (70) pour traiter lesdits signaux électriques en vue de réaliser ladite image radiographique comportent au moins l'un des éléments suivants : mémoire temporaire, mémoire permanente, convertisseur de signaux électriques en signaux vidéo aptes à être visualisés sur un écran.

12. Dispositif selon l'une des revendications 5 à 11, ***caractérisé par le fait que*** lesdits barreaux cylindriques (10) sont réalisés en cristal de iodure de césium.

13. Dispositif selon la revendication 12, ***caractérisé par le fait que*** lesdits barreaux cylindriques (10) sont sensiblement de forme cylindrique de révolution d'une longueur comprise entre 80 et 200 µm, pour un diamètre compris entre 3 et 7 µm.

14. Dispositif selon l'une des revendications 5 à 13, ***caractérisé par le fait que*** lesdits barreaux cylindriques (10) sont disposés au contact les uns des autres pour former une mosaïque.

## Claims

1. Method for obtaining a radiographic image of a tooth and its environment, consisting in:
emitting a beam of X rays in the direction of said tooth and its environment,
guiding the X rays emerging from said tooth and from its environment, in substantially cylindrical volumes, substantially along the axis of said volumes,
transforming the X rays as they are guided in said cylindrical volumes into light waves of greater wavelength than the X rays,
guiding said light rays in a bundle of optical fibres,
converting these light rays into electrical signals, and
processing these electrical signals to produce said radiographic image.

2. Method according to claim 1, **characterised in that** it further consists in filtering said electrical signals as a function of predetermined criteria.

3. Method according to either of the preceding claims, **characterised in that** said light rays are converted into analogue electrical signals and said analogue electrical signals are converted into digital electrical signals.

4. Method according to any one of the preceding claims, **characterized in that** it consists in amplifying at least a portion of said electrical signals in accordance with a predetermined function.

5. Device for implementing the method according to any one of the preceding claims, including:
a source (1) adapted to emit a beam of X rays toward said tooth (4) and its environment (5),
a rod produced in a material adapted to transform the X rays into light rays of greater wavelength than the X rays, the rod having an entry face adapted to receive said X rays and an exit face adapted to emit said light rays,
means (20) for converting light rays (11) into electrical signals,
means (30) for coupling the exit face (13) of the cylindrical rod with said means (20) for converting light rays (11) into electrical signals, and
means (70) for processing said electrical signals in order to produce said radiographic image,
**characterised in that** the device includes a plurality of cylindrical rods (10) produced in a material adapted to transform the X rays into light rays (11) of greater wavelength than the X rays, each rod having an entry face (12) adapted to receive said X rays and an exit face (13) adapted to emit said light rays (11), said cylindrical rods (10) being disposed side-by-side so that all the entry faces (12) face said source (1) of X rays,
the means (30) for coupling the exit faces (13) of the cylindrical rods with said means (20) for converting light rays into electrical signals including a bundle of optical fibres.

6. Device according to claim 5, **characterised in that** the means (20) for converting light rays into electrical signals include a converter (21) of light rays into analogue electrical signals and a converter (22) of analogue electrical signals into digital electrical signals the input (23) of which is connected to the output (24) of the converter of light rays into analogue electrical signals.

7. Device according to claim 6, **characterised in that** said converter (21) of light rays into analogue electrical signals consists of a CCD strip and said converter (22) of analogue electrical signals into digital electrical signals is an at least 12-bit ADC converter.

8. Device according to any one of claims 5 to 7, **characterised in that** it further includes means (40) for filtering said electrical signals.

9. Device according to claim 8, **characterised in that** said means (40) for filtering the electrical signals include at least one of the following three filters: a low-pass filter (41) adapted to eliminate the electrical signals corresponding to the X rays that have passed through the most opaque portions of said tooth (4) and its environment (5), a band-pass filter (42) adapted to pass the electrical signals corresponding to the X rays that have passed through the dentine of the tooth and the portions of matter of its environment substantially equivalent to that dentine, a high-pass filter (43) adapted to eliminate the electrical signals corresponding to the X rays that have passed through soft tissue or the like of the tooth and its environment.

10. Device according to claim 9, **characterised in that** it further includes means (50) for amplifying the electrical signals obtained at the output (44) of at least one of the three filters (41-43) in accordance with a predetermined function.

11. Device according to any one of claims 5 to 10, **characterised in that** the means (70) for processing said electrical signals in order to produce said radiographic image include at least one of the following elements: temporary memory, permanent memory, converter of electrical signals into video signals adapted to be viewed on a screen.

12. Device according to any one of claims 5 to 11, **characterised in that** said cylindrical rods (10) are produced in caesium iodide crystal.

13. Device according to claim 12, **characterised in that** said cylindrical rods (10) are of substantially circular cylindrical shape, from 80 to 200 µm long, with a diameter from 3 to 7 µm.

14. Device according to any one of claims 5 to 13, **characterised in that** said cylindrical rods (10) are disposed in contact with each other to form a mosaic.

## Patentansprüche

1. Verfahren zum Erhalten eines Röntgenbildes eines Zahns und seiner Umgebung, das darin besteht:
ein Röntgenstrahlenbündel in Richtung des Zahns und seiner Umgebung zu emittieren,
die Röntgenstrahlen, die aus dem Zahn und seiner Umgebung austreten, in im Wesentlichen zylindrischen Volumina im Wesentlichen längs der Achse dieser Volumina zu führen,
die Röntgenstrahlen bei ihrer Führung in den zylindrischen Volumina in Lichtstrahlen mit einer Wellenlänge größer als jene der Röntgenstrahlen zu transformieren,
die Lichtstrahlen in einem Lichtleitfaserbündel zu führen,
diese Lichtstrahlen in elektrische Signale umzusetzen und
diese elektrischen Signale zu verarbeiten, um das Röntgenbild zu erzeugen.

2. Verfahren nach Anspruch 1, **dadurch gekennzeichnet, dass** es außerdem darin besteht, die elektrischen Signale als Funktion vorgegebener Kriterien zu filtern.

3. Verfahren nach einem der vorhergehenden Ansprüche, **dadurch gekennzeichnet, dass** die Lichtstrahlen in analoge elektrische Signale umgesetzt werden und dass diese analogen elektrische Signale in digitale elektrische Signale umgesetzt werden.

4. Verfahren nach einem der vorhergehenden Ansprüche, **dadurch gekennzeichnet, dass** es darin besteht, wenigstens einen Teil der elektrischen Signale gemäß einer vorgegebenen Funktion zu verstärken.

5. Vorrichtung für die Ausführung des Verfahrens nach einem der vorhergehenden Ansprüche, die umfasst:
eine Quelle (1), die dazu ausgelegt ist, ein Röntgenstrahlbündel zu einem Zahn (4) und seiner Umgebung (5) zu emittieren,
einen Stab, der aus einem Material hergestellt ist, das die Röntgenstrahlen in Lichtstrahlen mit einer Wellenlänge größer als jene der Röntgenstrahlen transformieren kann, wobei der Stab eine Eintrittsfläche, die die Röntgenstrahlen empfangen kann, und eine Austrittsfläche, die die Lichtstrahlen emittieren kann, umfasst,
Mittel (20), um die Lichtstrahlen (11) in elektrische Signale umzusetzen,
Mittel (30), um die Austrittsfläche (13) des zylindrischen Stabs mit den Mitteln (20) zum Umsetzen der Lichtstrahlen (11) in elektrische Signale zu koppeln, und
Mittel (70), um die elektrischen Signale zu verarbeiten, um das Röntgenbild zu erzeugen,
**dadurch gekennzeichnet, dass** die Vorrichtung mehrere zylindrische Stäbe (10) umfasst, die aus einem Material hergestellt sind, das die Röntgenstrahlen in Lichtstrahlen (11) mit einer Wellenlänge größer als jene der Röntgenstrahlen transformieren kann, wobei jeder Stab eine Eintrittsfläche (12), die die Röntgenstrahlen empfangen kann, und eine Austrittsfläche (13), die die Lichtstrahlen (11) emittieren kann, umfasst, wobei die zylindrischen Stäbe (10) nebeneinander in der Weise angeordnet sind, dass alle Eintrittsflächen (12) zur Röntgenstrahlquelle (1) gerichtet sind,
wobei die Mittel (30) zum Koppeln der Austrittsflächen (13) der zylindrischen Stäbe mit den Mitteln (20) zum Umsetzen der Lichtstrahlen in elektrische Signale ein Lichtleitfaserbündel umfassen.

6. Vorrichtung nach Anspruch 5, **dadurch gekennzeichnet, dass** die Mittel (20) zum Umsetzen der Lichtstrahlen in elektrische Signale einen Umsetzer (21) von Lichtstrahlen in analoge elektrische Signale und einen Umsetzer (22) von analogen elektrischen Signalen in digitale elektrische Signale, dessen Eingang (23) mit dem Ausgang (24) des Umsetzers von Lichtstrahlen in analoge elektrische Signale verbunden ist, umfassen.

7. Vorrichtung nach Anspruch 6, **dadurch gekennzeichnet, dass** der Umsetzer (21) von Lichtstrahlen in analoge elektrische Signale aus einem CCD-Stab gebildet ist und dass der Umsetzer (22) von analogen elektrischen Signalen in digitale elektrische Signale ein CAN-Umsetzer des Typs mit wenigstens zwölf Bit ist.

8. Vorrichtung nach einem der Ansprüche 5 bis 7, **dadurch gekennzeichnet, dass** sie außerdem Mittel (40) umfasst, um die elektrischen Signale zu filtern.

9. Vorrichtung nach Anspruch 8, **dadurch gekennzeichnet, dass** die Mittel (40) zum Filtern der elektrischen Signale wenigstens eines der drei folgenden Filter umfassen: ein Tiefpassfilter (41), das jene elektrischen Signale beseitigen kann, die den Röntgenstrahlen entsprechen, die die optisch dichtesten Teile des Zahns (4) und seiner Umgebung (5) durchquert haben, ein Bandpassfilter (42), das jene elektrischen Signale durchlassen kann, die den Röntgenstrahlen entsprechen, die das Zahnbein des Zahns und die Materialteile seiner Umgebung, die zu diesem Zahnbein im Wesentlichen äquivalent sind, durchquert haben, und ein Hochpassfilter (43), das jene elektrischen Signale beseitigen kann, die den Röntgenstrahlen entsprechen, die die Weichgewebe oder Äquivalente des Zahns und seiner Umgebung durchquert haben.

10. Vorrichtung nach Anspruch 9, **dadurch gekennzeichnet, dass** sie außerdem Mittel (50) umfasst, um die elektrischen Signale, die am Ausgang (44) wenigstens eines der drei Filter (41-43) erhalten werden, gemäß einer vorgegebenen Funktion zu verstärken.

11. Vorrichtung nach einem der Ansprüche 5 bis 10, **dadurch gekennzeichnet, dass** die Mittel (70) zum Verarbeiten der elektrischen Signale, um das Röntgenbild zu erzeugen, wenigstens eines der folgenden Elemente umfassen: Zwischenspeicher, Festspeicher, und Umsetzer elektrischer Signale in Videosignale, die auf einem Bildschirm sichtbar gemacht werden können.

12. Vorrichtung nach einem der Ansprüche 5 bis 11, **dadurch gekennzeichnet, dass** die zylindrischen Stäbe (10) aus einem Cäsiumjodid-Kristall verwirklicht sind.

13. Vorrichtung nach Anspruch 12, **dadurch gekennzeichnet, dass** die zylindrischen Stäbe (10) eine im Wesentlichen rotationssymmetrische zylindrische Form haben, eine Länge im Bereich von 80 bis 200 µm haben und einen Durchmesser im Bereich von 3 bis 7 µm besitzen.

14. Vorrichtung nach einem der Ansprüche 5 bis 13, **dadurch gekennzeichnet, dass** die zylindrischen Stäbe (10) in gegenseitigem Kontakt angeordnet sind, um ein Mosaik zu bilden.
